(19) Europäisches Patentamt
European Patent Office
Office européen des brevets

(11) Veröffentlichungsnummer: **0 003 211**
**B1**

(12) # EUROPÄISCHE PATENTSCHRIFT

(45) Veröffentlichungstag der Patentschrift: **25.11.81**

(51) Int. Cl.³: **A 01 N 43/50,**
C 07 D 233/58

(21) Anmeldenummer: **78101256.2**

(22) Anmeldetag: **28.10.78**

(54) **Verfahren zur Bekämpfung von Insekten mit Imidazolderivaten.**

(30) Priorität: **09.11.77 DE 2750031**

(43) Veröffentlichungstag der Anmeldung:
**08.08.79 Patentblatt 79/16**

(45) Bekanntmachung des Hinweises auf die Patenterteilung:
**25.11.81 Patentblatt 81/47**

(84) Benannte Vertragsstaaten:
**BE DE FR GB NL**

(56) Entgegenhaltungen:
**DE - A - 2 442 706**
**DE - A - 2 510 525**
**DE - A - 2 706 838**
**FR - A - 1 519 132**
**GB - A - 1 364 312**
**US - A - 3 991 201**

(73) Patentinhaber: **BASF Aktiengesellschaft**
**Carl-Bosch-Strasse 38**
**D-6700 Ludwigshafen (DE)**

(72) Erfinder: **Goetz, Norbert, Dr.**
**Schoefferstrasse 25**
**D-6520 Worms (DE)**
Erfinder: **Steimmig, Anna, Dr.**
**Freinsheimer Strasse 9**
**D-6700 Ludwigshafen (DE)**
Erfinder: **Zeeh, Bernd, Dr.**
**Thorwaldsenstrasse 5**
**D-6700 Ludwigshafen (DE)**
Erfinder: **Adolphi, Heinrich, Dr.**
**Kalmitweg 11**
**D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 211 B1

# Verfahren zur Bekämpfung von Insekten mit Imidazolderivaten

Die vorliegende Erfindung betrifft ein Verfahren zur Bekämpfung von Insekten mit Imidazolderivaten oder deren Salzen.

Aus der britischen Patentschrift 1 148 103 ist bekannt, daß Imidazole und ihre Salze geeignete Synergisten für die Wirkungssteigerung von insektiziden Wirkstoffen, wie Pyrethrine, Carbamate und Phosphorsäureester, sind. Die dort beschriebenen Imidazole zeigen selbst keine oder nur eine sehr geringe insektizide Wirksamkeit gegenüber den Vertretern der Ordnung Coleoptera und Diptera, sondern wirken ausschließlich als Synergisten.

Außerdem ist aus der britischen Patentschrift 1 364 312 bekannt, daß Imidazolderivate, die in 1-Stellung einen langkettigen Alkyl-, Alkenyl- oder Alkinylrest tragen, zur Senkung des Cholesterinspiegels im Blut geeignet sind.

Es wurde gefunden, daß Imidazolderivate der Formel I

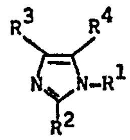

I,

in der

$R^1$ für verzweigte Alkylreste mit 6 bis 20 Kohlenstoffatomen, für unverzweigte Alkylreste von 6 bis 16 Kohlenstoffatomen, für verzweigte Alkenylreste mit 10 bis 20 Kohlenstoffatomen, für unverzweigte oder verzweigte Alkinylreste mit 3 bis 6 Kohlenstoffatomen, für 2-Methyl-3-phenyl-propyl- oder 2-Methyl-3-phenyl-propen-2-yl-reste, wobei die Phenylreste durch Halogen oder Alkylgruppen mit 1 bis 10 Kohlenstoffatomen einfach oder mehrfach substituiert sein können, und

$R^2$, $R^3$ und $R^4$ für Wasserstoff oder Methyl stehen, oder Salze dieser Imidazolderivate, sich zur Bekämpfung von Insekten aus der Klasse der Lepidoptera, Coleoptera, Diptera, Hymenoptera, Heteroptera, Homoptera und Isoptera eignen. Sie greifen in das hormonale System des tierischen Organismus ein, so daß die Umwandlung zur Imago, die Ablage von entwicklungsfähigen Eiern und die Entwicklung von abgelegten normalen Eiern gestört sind.

Wirksame Verbindungen dieser Art, die als Juvenoide bezeichnet werden, sind Imidazole der Formel I, bei denen $R^1$ verzweigte Alkylreste mit 6 bis 20 Kohlenstoffatomen, insbesondere mit 10 bis 20 Kohlenstoffatomen, unverzweigte Alkylreste mit 6 bis 16 Kohlenstoffatomen, insbesondere 10 bis 14 Kohlenstoffatomen, verzweigte Alkenylreste mit 10 bis 20 Kohlenstoffatomen, insbesondere 10 bis 15 Kohlenstoffatomen, oder unverzweigte oder verzweigte Alkinylreste mit 3 bis 6 Kohlenstoffatomen und $R^2$, $R^3$ und $R^4$ Wasserstoff oder Methyl bedeuten. Geeignete Juvenoide sind außerdem Imidazole, die nur in 1-Stellung substituiert sind und bei denen $R^1$ für 2-Methyl-3-phenyl-propyl- oder 2-Methyl-3-phenyl-propenylreste steht, bei denen der Phenylrest durch Halogen, insbesondere Chlor, oder Alkylreste mit 1 bis 10 Kohlenstoffatomen, insbesondere 1 bis 5 Kohlenstoffatomen, einfach oder mehrfach, vorzugsweise in 4- und 2,4-Stellung, substituiert sein kann.

Sehr wirksame Imidazole sind beispielsweise solche, bei denen $R^2$ Wasserstoff oder Methyl, $R^3$ und $R^4$ Wasserstoff und $R^1$ verzweigte Alkyl- oder Alkenylreste mit Isoprenstruktur und 10 bis 20 Kohlenstoffatomen, insbesondere 10 bis 15 Kohlenstoffatomen, wie 3,7-Dimethyloctyl, 1,5,9-Trimethyl-decyl, 3,7,11-Trimethyl-dodecyl, 3,7-Dimethyl-octa-2,6-dienyl, 3,7,11-Trimethyl-dodeca-2,6,10-trienyl, bedeuten. Außerdem sehr wirksam sind Imidazole, die in 1-Stellung unverzweigte Alkylreste mit 10 bis 14 Kohlenstoffatomen, wie n-Decyl, n-Dodecyl, n-Tetradecyl, in 2-Stellung Wasserstoff und in 4- und 5-Stellung Wasserstoff oder Methyl tragen.

Als Salze kommen Salze der Imidazolderivate mit anorganischen oder organischen Säuren in Betracht, beispielsweise mit Halogenwasserstoffsäuren, wie Chlorwasserstoffsäure, Bromwasserstoffsäure, mit Schwefelsäure, Salpetersäure, Phosphorsäure, Ameisensäure, Essigsäure, Propionsäure, Buttersäure, Arcylsäure, Oxalsäure, Adipinsäure, Milchsäure, Weinsäure, Citronensäure, Trichloressigsäure, Stearinsäure, Ölsäure, Phenol, Perfluoroctansäure, p-Toluolsulfonsäure und Dodecylbenzolsulfonsäure.

Die Herstellung der substituierten Imidazole kann durch Ringschlußsynthesen von Imidazolen nach bekannten Methoden, durch Alkylierung von Imidazol mit den entsprechenden Alkoholen an Dehydratisierungskatalysatoren oder mit gegebenenfalls substituierten Alkyl-, Alkenyl- und Alkinylhalogeniden erfolgen.

Die folgenden Beispiele erläutern die Herstellung der Imidazolderivate.

## Beispiel 1

Herstellung von 1-(1,5,9-Trimethyl-decyl)-imidazol

52,6 Gewichtsteile 6,10-Dimethyl-undec-2-yl-bromid und 27,2 Gewichtsteile Imidazol werden in 80 Gewichtsteilen Tetrahydrofuran gelöst, in einen 250 ml Autoklaven gefüllt und 15 Stunden lang auf

160°C erhitzt. Das Reaktionsprodukt wird anschließend filtriert und aus dem Filtrat bei Normaldruck das Lösungsmittel entfernt. Die Destillation des hierbei erhaltenen Rückstandes unter Vakuum ergibt 41,5 Gewichtsteile 1-(1,5,9-Trimethyl-decyl)-imidazol; Kp = 185°C. Ausbeute: 83% der Theorie.

Beispiel 2

Herstellung von 1-(3,7,11-Trimethyl-dodeca-2,6,10-trienyl)-imidazol

72,3 Gewichtsteile 3,7,11-Trimethyl-dodeca-2,6,10-trienyl-chlorid und 40,8 Gewichtsteile Imidazol werden in 500 Gewichtsteilen Dioxan gelöst. Anschließend wird das Reaktionsgemisch 6 Stunden lang unter Rückfluß erhitzt. Nach dem Abkühlen wird filtriert, und das Filtrat wird unter Vakuum eingeengt. Die Destillation des Rückstandes aus dem Filtrat im Ölpumpenvakuum ergibt 72 Gewichtsteile 1-(3,7,11-Trimethyl-dodeca-2,6,10-trienyl)-imidazol; $Kp_{0,01}$ = 155°C; Ausbeute: 88% der Theorie.

Erfindungsgemäß zu verwendende Imidazole sind beispielsweise Imidazole der Formel I, in der die Substituenten $R^1$, $R^2$, $R^3$ und $R^4$ die folgenden Bedeutungen haben:

| Nr. | $R^1$ | $R^2$ | $R^3$ | $R^4$ | Kp(°C)/Torr |
|---|---|---|---|---|---|
| 1 | 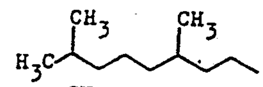 | H | H | H | 118/0,01 |
| 2 | 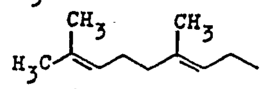 | H | H | H | 124/0,01 |
| 3 | 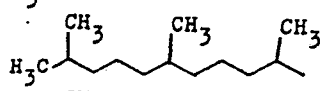 | H | H | H | 185/4 |
| 4 | 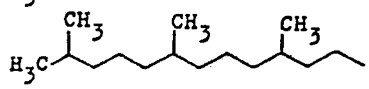 | H | H | H | 140/0,01 |
| 5 | 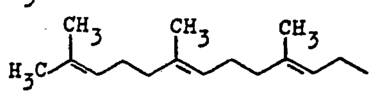 | H | H | H | 155/0,01 |
| 6 | 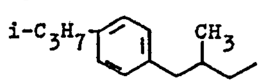 | H | H | H | 144/0,1 |
| 7 | 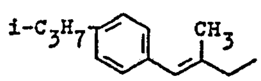 | H | H | H | 158/0,2 |
| 8 | 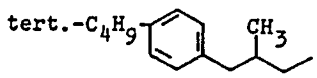 | H | H | H | 155/0,1 |
| 9 | 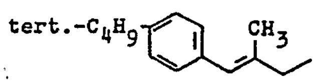 | H | H | H | 169/0,1 |
| 10 | 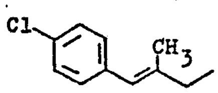 | H | H | H | 165/0,1 |
| 11 | 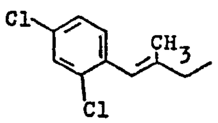 | H | H | H | 164/1 |
| 12 | $n-C_{14}H_{29}$ | H | H | H | 164/1 |
| 13 | $n-C_{13}H_{27}$ | H | H | H | 170/4 |
| 14 | $n-C_{12}H_{25}$ | H | H | H | 149—152/1 |
| 15 | $n-C_{10}H_{21}$ | H | H | H | 135/2 |
| 16 | $C_9H_{19}$ | H | H | H | 175/12 |
| 17 | $n-C_{12}H_{25}$ | H | $CH_3$ | $CH_3$ | 170/2 |
| 18 | $CH_3—C(CH_3)_2—CH_2—CH_2—$ | H | H | H | 121/9 |

| Nr. | $R^1$ | $R^2$ | $R^3$ | $R^4$ | Kp(°C)/Torr |
|---|---|---|---|---|---|
| 19 | $HC{\equiv}C{-}CH_2{-}$ | H | H | H | 76—78/0,6 |
| 20 | 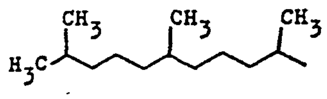 | $CH_3$ | H | H | 126/0,1 |
| 21 | $HC{\equiv}C{-}CH(CH_3){-}$ | H | H | H | |
| 22 | $H_3C{-}C{\equiv}C{-}CH_2{-}$ | H | H | H | |
| 23 | $H_3C{-}CH_2{-}CH_2{-}CH(C_2H_5){-}$ | H | H | H | |
| 24 | $H_3C{-}(CH_2)_4{-}CH(CH_3){-}$ | H | H | H | |
| 25 | $H_3C{-}CH(CH_3){-}CH_2{-}CH(i\text{-}C_4H_9){-}$ | H | H | H | |
| 26 | $H_3C{-}CH_2{-}CH_2{-}CH(n\text{-}C_3H_7){-}$ | H | H | H | |
| 27 | $H_3C{-}(CH_2)_3{-}CH(CH_3){-}CH(CH_3){-}$ | H | H | H | |
| 28 | $H_3C{-}CH_2{-}CH(C_2H_5){-}(CH_2)_2{-}CH(CH_3){-}$ | H | H | H | |
| 29 | $H_3C{-}(CH_2)_3{-}CH(C_2H_5){-}$ | H | H | H | |
| 30 | $H_3C{-}(CH_2)_5{-}CH(CH_3){-}$ | H | H | H | |
| 31 | $H_3C{-}CH_2{-}CH(C_2H_5){-}(CH_2)_2{-}CH(C_2H_5){-}$ | H | H | H | |
| 32 | $H_3C{-}(CH_2)_3{-}CH(C_2H_5){-}(CH_2)_2{-}CH(CH_3){-}$ | H | H | H | |
| 33 | $H_3C{-}CH(CH_3){-}CH_2{-}CH(CH_3){-}CH_2{-}CH(i\text{-}C_4H_9){-}$ | H | H | H | |
| 34 | $H_3C{-}CH_2{-}CH(C_2H_5){-}(CH_2)_2{-}CH(i\text{-}C_4H_9){-}$ | H | H | H | |
| 35 | $H_3C{-}(CH_2)_3{-}CH(C_2H_5){-}(CH_2)_2{-}CH(C_2H_5){-}$ | H | H | H | |
| 36 | $H_3C{-}(CH_2)_3{-}CH(C_2H_5){-}(CH_2)_2{-}CH(i\text{-}C_4H_9){-}$ | H | H | H | |
| 37 | $H_3C{-}C(CH_3)_2{-}CH_2{-}CH(CH_3){-}(CH_2)_2{-}$ | H | H | H | |
| 38 | $H_3C{-}(CH_2)_3{-}CH(CH_3){-}$ | H | H | H | |
| 39 | $H_3C{-}(CH_2)_4{-}CH(CH_3){-}$ | H | H | H | |
| 40 | $H_3C{-}(CH_2)_9{-}CH(CH_3){-}$ | H | H | H | |
| 41 | $H_3C{-}(CH_2)_2{-}CH(n\text{-}C_3H_7){-}$ | H | H | H | |
| 42 | $H_3C{-}(CH_2)_8{-}CH(CH_3){-}$ | H | H | H | |
| 43 | $n\text{-}C_6H_{13}$ | H | H | H | |
| 44 | $n\text{-}C_7H_{15}$ | H | H | H | |
| 45 | $n\text{-}C_8H_{17}$ | H | H | H | |
| 46 | $n\text{-}C_{11}H_{23}$ | H | H | H | |
| 47 | $n\text{-}C_{16}H_{33}$ | H | H | H | |
| 48 | $H_3C{-}(CH_2)_5{-}CH(C_4H_9){-}CH_2{-}$ | H | H | H | |

| Nr. | $R^1$ | $R^2$ | $R^3$ | $R^4$ | Kp(°C)/Torr |
|---|---|---|---|---|---|
| 49 | $H_3C—(CH_2)_9—CH(CH_3)—CH_2—$ | H | H | H | |
| 50 | $H_3C—CH(CH_3)—CH_2—C(CH_3)_2—CH_2—$ | H | H | H | |
| 51 | $H_3C—(CH_2)_3—CH(C_2H_5)—CH_2—$ | H | H | H | |

Die Imidazolderivate der Formel I

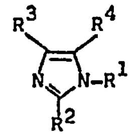

in der

$R^1$ für 2-Methyl-3-phenyl-propyl- oder 2-Methyl-3-phenyl-propen-2-yl-reste, wobei die Phenylreste durch Halogen oder Alkylgruppen mit 1 bis 10 Kohlenstoffatomen einfach oder mehrfach substituiert sein können,

$R^2$ für Wasserstoff oder Methyl und

$R^3$ und $R^4$ für Wasserstoff stehen, sind neu.

Die Juvenoide der Formel I eignen sich als Entwicklungshemmer und Ovizide zur Bekämpfung von Insekten der Klassen Lepidopteren, Coleopteren, Dipteren, Hymenopteren, Heteropteren, Homopteren und Isopteren, vorzugsweise während des Embryonal-, Larven- oder Puppenstadiums.

Die Wirkstoffe können mit Erfolg im Pflanzenschutz sowie bei der Bekämpfung von krankheitsübertragenden Insekten eingesetzt werden, wobei man sie auf die Insekten bzw. deren Lebensraum einwirken läßt. Der Einsatz erfolgt im allgemeinen ebenso wie bei den üblichen Kontaktinsektiziden.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz sowie Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:
Alkali-, Erdalkali-, Ammoniumsalze von Ligninsulfonsäure, Naphthalinsulfonsäure, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenolpolyglykoläther, Alkylarylpolyätheralkohole, Isotridecylalkohol, Fettalkoholäthylenoxid-Kondensate, äthoxyliertes Rizinusöl, Polyoxyäthylalkyläther, äthoxyliertes Polyoxypropylen, Laurylalkoholpolyglykolätheracetal, Sorbitester, Lignin, Sulfitablaugen und Methylcellulose.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß,

Ton, Dolomit, Diatomeenerde, Kalcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten im allgemeinen zwischen 0,1 und 95 Gewichtsprozent Wirkstoff, vorzugsweise zwischen 0,5 und 90 Gewichtsprozent.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gewichtsprozent Wirkstoff oder sogar den reinen Wirkstoff auszubringen.

Beispiele für Formulierungen sind:

I. 3 Gewichtsteile 1-(3,7,11-Trimethyl-dodeca-2,6,10-trienyl)-imidazol werden mit 97 Gewichtsteilen feinteiligem Kaolin innig vermischt. Man erhält auf diese Weise ein Stäubemittel, das 3 Gew.% des Wirkstoffs enthält.

II. 20 Teile 1-(3,7-Dimethyl-octa-2,6-dienyl)-imidazol werden mit 2 Teilen Calciumsalz der Dodecylbenzolsulfonsäure, 8 Teile Fettalkohol-polyglykoläther, 2 Teile Natriumsalz eines Phenolsulfonsäure-harnstoff-formaldehyd-kondensats und 68 Teile eines paraffinischen Mineralöls innig vermischt. Man erhält eine stabile ölige Dispersion.

III. 20 Gewichtsteile 1 - [3 - (4 - Isopropylphenyl) - 2 - methylpropen - 2 - yl] - imidazol werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile 1-(Propin-2-yl)-imidazol werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Zu den Wirkstoffen können Öle verschiedenen Typs, Herbizide, Fungizide, andere Insektizide, Insektenlockstoffe, Bakterizide, gegebenenfalls auch erst unmittelbar vor der Anwendung (Tankmix), zugesetzt werden. Diese Mittel können zu den erfindungsgemäßen Wirkstoffen im Gewichtsverhältnis 1:10 bis 10:1 zugemischt werden.

Beispielsweise können folgende Mittel zugemischt werden:

2 - sec - Butyl - phenyl - N - methylcarbamat, o - Chlorphenyl - N - methylcarbamat, 3 - Isopropyl - 5 - methyl - phenyl - N - methylcarbamat, o - Isopropoxyphenyl - N - methylcarbamat, 3,5 - Dimethyl - 4 - methylmercapto - phenyl - N - methylcarbamat, 4 - Dimethylamino - 3,5 - xylyl - N - methylcarbamat, 2 - (1,3 - Dioxolan - 2 - yl) - phenyl - N - methylcarbamat, 1 - Naphthyl - N - methylcarbamat, 2,3 - Dihydro - 2,2 - dimethyl - benzofuran - 7 - yl - N - methylcarbamat, 2,2 - Dimethyl - 1,3 - benzodioxol - 4 - yl - N - methylcarbamat, 2 - Dimethylamino - 5,6 - dimethyl - 4 - pyrimidinyl - dimethylcarbamat, 2 - Methyl - 2 - (methylthio) - propionaldehyd - O - (methylcarbamoyl) - oxim, S - Methyl - N - [(methylcarbamoyl)oxy] - thioacetimidat, Methyl - N',N' - dimethyl - N - [(methylcarbamoyl)oxy] - 1 - thioxamimidat, N - (2 - Methyl - 4 - chlor - phenyl) - N',N' - dimethyl - formamidin, Tetrachlorthiophen, O,O - Dimethyl - O - (p - nitrophenyl) - phosphorthioat, O,O - Diäthyl - O - (p - nitrophenyl) - phosphorthioat, O - Äthyl - O - (p - nitrophenyl) - phenyl - phosphonothioat, O,O - Dimethyl - O - (3 - methyl - 4 - nitrophenyl) - phosphorthioat, O,O - Diäthyl - O - (2,4 - dichlorphenyl) - phosphorthioat, O - Äthyl - O - (2,4 - dichlorphenyl) - phenyl - phosphonothioat, O,O - Dimethyl - O - (2,4,5 - trichlorphenyl) - phosphorthioat, O - Äthyl - O - (2,4,5 - trichlorphenyl) - äthyl - phosphonothioat, O,O - Dimethyl - O - (4 - brom - 2,5 - dichlorphenyl) - phosphorthioat, O,O - Dimethyl - O - (2,5 - dichlor - 4 - jodphenyl) - phosphorthioat, O,O - Dimethyl - O - (3 - methyl - 4 - methylthiophenyl) - phosphorthioat, O - Äthyl - O - (3 - methyl - 4 - methylthiophenyl) - isopropyl - phosphoramidat, O,O - Diäthyl - O - [p - (methylsulfinyl)phenyl] - phosphorthioat, O - Äthyl - S - phenyl - äthyl - phosphonodithioat, O,O - Diäthyl - [2 - chlor - 1 - (2,4 - dichlorphenyl) - vinyl] - phosphat, O,O - Dimethyl - [2 - chlor - 1 - (2,4,5 - trichlorphenyl)] - vinyl - phosphat, O,O - Dimethyl - S - (1' - phenyl) - äthylacetat - phosphordithioat, Bis - (dimethylamino) - fluor - phosphinoxid, Octamethyl - pyro - phosphoramid, O,O,O,O - Tetraäthyl - dithio - pyrophosphat, S - Chlormethyl - O,O - diäthyl - phosphordithioat, O - Äthyl - S,S - di - n - propyl - phosphordithioat, O,O - Dimethyl - O - 2,2 - dichlorvinyl - phosphat, O,O - Dimethyl -

1,2 - dibrom - 2,2 - dichloräthyl - phosphat, O,O - Dimethyl - 2,2,2 - trichlor - 1 - hydroxy - äthyl - phosphat, O,O - Dimethyl - S - [1,2 - biscarbäthoxy - äthyl - (1)] - phosphordithioat, O,O - Dimethyl - O - (1 - methyl - 2 - carbmethoxy - vinyl) - phosphat, O,O - Dimethyl - S - (N - methyl - carbamoyl - methyl) - phosphordithioat, O,O - Dimethyl - S - (N - methyl - carbamoyl - methyl) - phosphorthioat, O,O - Dimethyl - S - (N - methoxy - äthyl - carbamoyl - methyl) - phosphordithioat, O,O - Dimethyl - S - (N - formyl - N - methyl - carbamoylmethyl) - phosphordithioat, O,O - Dimethyl - O - [1 - methyl - 2 - (methyl - carbamoyl) - vinyl] - phosphat, O,O - Dimethyl - O - [(1 - methyl - 2 - dimethylcarbamoyl) - vinyl] - phosphat, O,O - Dimethyl - O - [(1 - methyl - 2 - chlor - 2 - diäthyl - carbamoyl) - vinyl] - phosphat, O,O - Diäthyl - S - (äthylthio - methyl) - phosphordithioat, O,O - Diäthyl - S - [(p - chlorphenylthio) - methyl] - phosphordithioat, O,O - Dimethyl - S - (2 - äthylthioäthyl) - phosphorthioat, O,O - Dimethyl - S - (2 - äthylthioäthyl) - phosphordithioat, O,O - Dimethyl - S - (2 - äthylsulfinyl - äthyl) - phosphorthioat, O,O-Diäthyl - S - (2 - äthylthio - äthyl) - phosphordithioat, O,O - Dimethyl - S - (2 - äthylsulfinyl - äthyl)-phosphorthioat, O,O - Diäthyl - thiophosphoryl - iminophenyl - acetonitril, O,O - Diäthyl - S - (2 - chlor-1 - phthalimidoäthyl) - phosphordithioat, O,O - Diäthyl - S - [6 - chlor - benzoxazolon - (2) - yl - (3)] - methyldithiophosphat, O,O - Dimethyl - S - (2 - methoxy - 1,3,4 - thiadiazol - 5 - [4H] - onyl - (4) - methyl) - phosphordithioat, O,O - Diäthyl - O - [3,5,6 - trichlor - pyridyl - (2)] - phosphorthioat, O,O - Diäthyl - O - (2 - pyrazinyl) - phosphorthioat, O,O - Diäthyl - O - [2 - isopropyl - 4 - methyl - pyrimidinyl - (6)] - phosphorthioat, O,O - Diäthyl - O - [2 - (diäthylamino) - 6 - methyl - pyrimidin - 4 - yl] - thionophosphat, O,O - Dimethyl - S - (4 - oxo - 1,2,3 - benzotriazin - 3[4H] - yl - methyl) - phosphordithioat, O,O - Dimethyl - S - [4,6 - diamino - 1,3,5 - triazin - 2 - yl) - methyl] - phosphordithioat, O,O - Diäthyl - (1 - phenyl - 1,2,4 - triazol - 3 - yl) - thionophosphat, O,S - Dimethyl - phosphor - amido - thioat, O,S - Dimethyl - N -acetyl - phosphor amidothioat, $\gamma$ - Hexachlorcyclohexan, 1,1 - Di - (p - methoxyphenyl) - 2,2,2 - trichlor - äthan, 6,7,8,9,10,10 - Hexachloro - 1,5,5a,6,9,9a - hexahydro - 6,9 - methano - 2,4,3 - benzodioxathiepin - 3 - oxid.

Die folgenden Beispiele belegen die biologische Wirkung der Imidazolderivate der Formel I. Vergleichsmittel ist Äthyl-3,7,11-trimethyl-dodeca-2,4-dienoat (V) (DE—OS 22 02 016). Die Numerierung der Wirkstoffe entspricht der tabellarischen Aufzählung.

Beispiel A

Mehlkäfer (Tenebrio molitor); Puppentest

Maximal 24 Stunden alten Puppen des Mehlkäfers Tenebrio molitor werden mit einer Mikrometerspritze 2 ml der acetonischen Wirkstofflösung auf die Grenze zwischen Thorax und Abdomen gespritzt. Nach der Behandlung werden die Versuchstiere in Petrischalen von 10 cm Durchmesser auf Rundfilter gelegt.

Die Wirkung der Wirkstoffe wird danach beurteilt, wieviel Prozent der Tiere nach der Häutung starke Deformationen der Imaginal-Cuticula zeigen oder keine Metamorphose zum Käfer eingehen.

| Wirkstoff Nr. | Wirkstoffmenge pro Tier [$\mu$g] | Tiere mit Deformationen der Imaginal-Cuticula |
|---|---|---|
| 1 | 20 | 80 |
| 2 | 20 | 100 |
| | 2 | 40 |
| 3 | 20 | 50 |

Beispiel B

Wirkung auf Eier der Baumwollwanze (Dysdercus intermedius)

Ungefähr 200 frisch abgelegte Eier der Baumwollwanze werden auf Klebestreifen geheftet, die dann in die wäßrige Wirkstoffaufbereitung getaucht werden. Daraufhin lagert man die Streifen bei 25°C und 70% rel. Luftfeuchtigkeit bis zum Schlüpfen von unbehandelten Eiern.

| Wirkstoff Nr. | Wirkstoffkonzentration der wäßrigen Aufbereitung [Gew.%] | Mortalitätsrate [%] |
|---|---|---|
| 9 | 0,05 | 80 |
| 20 | 0,1 | 100 |
| V | 0,1 | unwirksam |

Beispiel C

Zuchtversuch mit Baumwollwanzen (Dysdercus intermedius)

In einem 1-1-Glas mit 300 g sterilisiertem angefeuchteten Sand züchtet man je 20 Larven von Dysdercus intermedius ab dem 4. Larvenstadium. Sie erhalten als Futter in Wasser gequollene Baumwollsamen. In den ersten beiden Wochen wird dem hierzu verwendeten Wasser der Wirkstoff zugesetzt, abschließend erfolgt Fütterung mit unbehandelter Baumwollsaat. Beobachtet werden die Metamorphose und die Eiablage.

| Wirkstoff Nr. | Wirkstoffkonzentration in dem zum Quellen der Baumwollsamen verwendeten Wasser [Gew.%] | |
|---|---|---|
| 13 | 0,1 | keine schlupffähigen Eier |
| 16 | 0,1 | keine schlupffähigen Eier |
| 18 | 0,1 | keine schlupffähigen Eier |
| 19 | 0,1 | keine schlupffähigen Eier |

Ein entsprechender Versuch, bei dem das Futter unbehandelt bleibt, der Sand jedoch mit dem Wirkstoff behandelt wird, bringt folgendes Ergebnis:

| Wirkstoff Nr. | Wirkstoffkonzentration im Sand [ppm] | |
|---|---|---|
| 1 | 10 | keine schlupffähigen Eier |
| 2 | 5 | keine schlupffähigen Eier |
| 3 | 25 | keine schlupffähigen Eier |
| 7 | 25 | keine schlupffähigen Eier |

Beispiel D

Zuchtversuch mit Moskito-Larven (Aedes aegypti)

In 200 ml Leitungswasser, das mit der Wirkstoffaufbereitung versetzt wurde, werden 30 bis 40 Aedes-Larven im 4. Stadium gebracht.

Die Versuchstemperatur beträgt 25°C. Beurteilt wird Verpuppung und Schlüpfen der Imagines, wobei eine unbehandelte Kontrolle als Maßstab dient. Während der Versuchsdauer wird einmal mit einem herkömmlichen pulverisierten Fischfutter gefüttert.

| Wirkstoff Nr. | Wirkstoffkonzentration im Leitungswasser [ppm] | |
|---|---|---|
| 12 | 0,1 | keine Imagines |
| 14 | 0,25 | keine Imagines |
| 15 | 2,0 | keine Imagines |
| 17 | 2,0 | keine Imagines |
| V | 2,0 | keine Imagines |

**Patentansprüche**

1. Verfahren zur Bekämpfung von Insekten, dadurch gekennzeichnet, daß man Imidazolderivate der Formel I

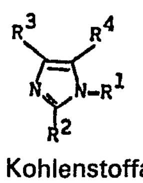

I,

in der

$R^1$ für verzweigte Alkylreste mit 10 bis 20 Kohlenstoffatomen der Formel

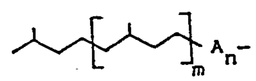

oder für verzweigte Alkenylreste mit 10 bis 20 Kohlenstoffatomen der Formel

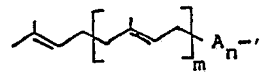

wobei m 1, 2 oder 3, A den bivalenten Rest —$CH_2$—$CH(CH_3)$— und n 0 oder 1 bedeuten, für 2-Methyl-3-phenyl-propyl- oder 2-Methyl-3-phenyl-propen-2-yl-reste, wobei die Phenylreste durch Halogen oder Alkylgruppen mit 1 bis 10 Kohlenstoffatomen einfach oder mehrfach substituiert sein können. und $R^2$, $R^3$ und $R^4$ für Wasserstoff oder Methyl stehen, oder Salze dieser Imidazolderivate auf die Insekten bzw. deren Lebensraum einwirken läßt.

2. Verfahren nach Anspruch 1, dadurch gekennzeichnet, daß man Imidazolderivate der Formel I oder Salze dieser Imidazolderivate auf die Insekten während des Embryonal-, Larven- oder Puppenstadiums einwirken läßt.

3. Verfahren nach Anspruch 1, dadurch gekennzeichnet, daß man Imidazolderivate der Formel I, in der $R^1$ für 2-Methyl-3-phenyl-propyl- oder 2-Methyl-3-phenyl-propen-2-yl-reste, wobei die Phenylreste durch Halogen oder Alkylgruppen mit 1 bis 10 Kohlenstoffatomen einfach oder mehrfach substituiert sein können, und $R^2$, $R^3$ und $R^4$ für Wasserstoff oder Methyl stehen, verwendet.

**Claims**

1. A process for controlling insects, characterized in that an imidazole derivative of the formula I

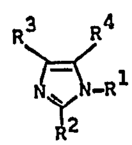 I,

where $R^1$ denotes branched alkyl of 10 to 20 carbon atoms of the formula

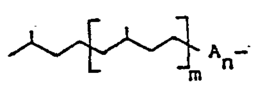

or branched alkenyl of 10 to 20 carbon atoms of the formula

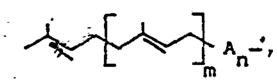

where m denotes 1, 2 or 3, A denotes the bivalent radical —$CH_2$—$CH(CH_3)$— and n denotes 0 or 1, $R^1$ further denotes 2-methyl-3-phenylpropyl or 2-methyl-3-phenylpropen-2-yl, phenyl being unsubstituted or mono- or polysubstituted by halogen or alkyl of 1 to 10 carbon atoms, and $R^2$, $R^3$ and $R^4$ denote hydrogen or methyl, or a salt of such an imidazole derivative, is allowed to act on the insects or their habitat.

2. A process as claimed in claim 1, characterized in that an imidazole derivative of the formula I, or a salt of such an imidazole derivative, is allowed to act on the insects during the embryonal, larval or pupal stage.

3. A process as claimed in claim 1, characterized in that there is used an imidazole derivative of the formula I where $R^1$ denotes 2-methyl-3-phenylpropyl or 2-methyl-3-phenylpropen-2-yl, phenyl being unsubstituted or mono- or polysubstituted by halogen or alkyl of 1 to 10 carbon atoms, and $R^2$, $R^3$ and $R^4$ denote hydrogen or methyl.

**Revendications**

1. Procédé de lutte contre les insectes, caractérisé par le fait que l'on fait agir sur les insectes, ou sur leur biotope, des dérivés (ou leurs sels) d'imidazole de formule I

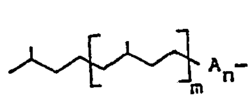

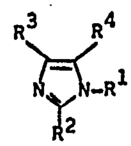

I,

dans laquelle

$R^1$ représente des restes ramifiés d'alkyle en $C_{10}$ à $C_{20}$ de formule

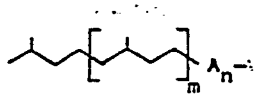

ou des restes ramifiés d'alcényle en $C_{10}$ à $C_{20}$ de formule

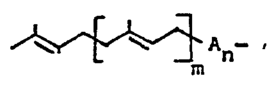

m représentant 1, 2 ou 3, A le reste bivalent $—CH_2CH(CH_3)—$ et n = 0 ou 1 des restes 2-méthyl-3-phényl-propyl- ou 2-méthyl-3-phényl-propène-2-yle, les restes phényle pouvant être substitués une ou plusieurs fois par un halogène ou des groupes alkyle en $C_1$ à $C_{10}$, et $R^2$, $R^3$ et $R^4$ représentent l'hydrogène ou le méthyle.

2. Procédé selon la revendication 1, caractérisé par le fait que l'on fait agir des dérivés d'imidazole de formule I, ou des sels de ces dérivés d'imidazole, sur les insectes pendant leur stade d'embryon, de larve ou de nymphe.

3. Procédé selon la revendication 1, caractérise par le fait que l'on utilise des dérivés d'imidazole de formule I, dans laquelle $R^1$ représente le reste 2-méthyl-3-phényl-propyle ou 2-méthyl-3-phényl-propèn-2-yle, les restes phényle pouvant être substitués une ou plusieurs fois par un halogène ou des groupes alkyle en $C_1$ à $C_{10}$, et $R^2$, $R^3$ et $R^4$ représentent l'hydrogène ou le méthyle.